# EUROPÄISCHE PATENTSCHRIFT

(11) **EP 3 248 571 B1**
(45) Veröffentlichungstag und Bekanntmachung des Hinweises auf die Patenterteilung: **01.01.2020**
(21) Anmeldenummer: 17169844.2
(22) Anmeldetag: 08.05.2017
(51) Int. Cl.: A61C 19/00, A61B 90/70

(54) **ANSCHLUSSADAPTER**
CONNECTION ADAPTER
ADAPTATEUR DE RACCORDEMENT

(30) Priorität: 25.05.2016 DE 102016109638
(43) Veröffentlichungstag der Anmeldung: 29.11.2017
(73) Patentinhaber: Miele & Cie. KG, 33332 Gütersloh (DE)
(72) Erfinder: Crilley, Ivana, 33335 Gütersloh (DE); Neufeld, Roman, 33739 Bielefeld (DE)

(56) Entgegenhaltungen:
- EP-A1- 2 279 708
- DE-A1- 10 241 542
- DE-B4- 19 626 871
- JP-A- H0 956 733

## Beschreibung

Die Erfindung betrifft einen Anschlussadapter für den strömungstechnischen Anschluss eines Spülguts, insbesondere eines dentalen Spülguts, an einen Anschlussstutzen einer der Zuführung einer Spülflüssigkeit dienenden Rohrleitung.

Zur Innenreinigung von insbesondere dentalem Spülgut, wie zum Beispiel Ultraschallspitzen, ist es aus dem Stand der Technik bekannt, eine mit Anschlussstutzen ausgerüstete Rohrleitung zu verwenden, an die das Spülgut zwecks Reinigung anzuschließen ist. Im bestimmungsgemäßen Verwendungsfall gelangt über die Rohrleitung Spülflüssigkeit zu den einzelnen Anschlussstutzen der Rohrleitung, von wo aus dann eine Beaufschlagung der an die Anschlussstutzen jeweils angeschlossenen Spülgüter stattfindet. Dabei ist typischerweise je zu reinigendem Spülgut ein Anschlussstutzen der Rohrleitung vorgesehen.

Die Anschlussstutzen sind standardisiert ausgebildet und verfügen typischerweise über ein Anschlussgewinde, beispielsweise in der Größe M8. Die zu reinigenden Spülgüter sind indes in ihrer Anschlussgeometrie unterschiedlich ausgebildet, je nach Hersteller und/oder Spülgut. Für einen bestimmungsgemäßen Anschluss eines Spülguts an einen Anschlussstutzen bedarf es daher der Zwischenschaltung eines Anschlussadapters, der einerseits eine Verbindungsgeometrie zum Anschlusstutzen sowie andererseits eine Verbindungsgeometrie zum zu reinigenden Spülgut bereitstellt. Im bestimmungsgemäßen Verwendungsfall ist der Anschlussadapter einerseits mit dem Anschlussstutzen der Rohrleitung und andererseits mit dem Spülgut verschraubt, so dass eine Innenbeaufschlagung des Spülguts mit Spülflüssigkeit stattfinden kann, die unter Zwischenschaltung des Anschlussadapters aus der Rohrleitung in das zu reinigende Spülgut einströmt.

Die DE 19626871 B4 beschreibt einen als mehrteiliger Halter ausgebildeten Anschlussadapter mit einem Anschlussteil, einem austauschbaren, und dabei jeweils an die Größe des zu reinigenden Instruments anzupassenden Kupplungsteil, welches eine Einstecköffnung zur flüssigkeitsdichten Instrumentenaufnahme aufweist, und einem kappenähnlichen Oberteil, mit dem das Kupplungsteil an dem Anschlussteil gehalten ist. Die DE 10241542 A1 beschreibt einen Aufsatz für Spraydosen zum Reinigen von einen Durchtrittskanal aufweisenden medizinischen Utensilien, welcher eine Eingriffskammer und eine daran anschließende Filterkammer aufweist.

Obgleich sich aus dem Stand der Technik vorbekannte Anschlussadapter im alltäglichen Praxiseinsatz bewährt haben, besteht Verbesserungsbedarf. Dabei ist insbesondere von Nachteil, dass je nach zu reinigendem Spülgut entsprechende Anschlussadapter verwenderseitig einzusetzen sind, was einerseits eine verwenderseitige Bevorratung von entsprechend vielen unterschiedlichen Anschlussadaptern erforderlich macht sowie andererseits die Handhabung aufgrund der unterschiedlich ausgestalteten Anschlussadapter aufwendig macht.

Es ist deshalb ausgehend vom Vorbeschriebenen die **Aufgabe** der Erfindung, einen Anschlussadapter bereitzustellen, der bei gleichzeitig vereinfachter Handhabung universeller einsetzbar ist.

Zur **Lösung** dieser Aufgabe wird mit der Erfindung ein Anschlussadapter mit den Merkmalen von Anspruch 1 vorgeschlagen.

Der erfindungsgemäße Anschlussadapter verfügt über einen Grundkörper einerseits und ein Verschlussteil andererseits. Dabei stellt der Grundkörper eine Spülkammer bereit, in die im bestimmungsgemäßen Verwendungsfall das zu reinigende Spülgut zumindest teilweise eingesetzt ist. Zwecks Beschickung der Spülkammer mit einem Spülgut ist eine Beschickungsöffnung vorgesehen. Durch diese hindurch ist das Spülgut verwenderseitig in die Spülkammer einzuführen.

Am Grundkörper bewegbar angeordnet ist ein Verschlussteil vorgesehen. Dieses Verschlussteil kann relativ gegenüber dem Grundkörper bewegt werden, was es gestattet, das Verschlussteil aus einer die Beschickungsöffnung verschließenden Verschlussstellung in eine die Beschickungsöffnung freigebende Beschickungsstellung zu bewegen und umgekehrt.

Der erfindungsgemäß ausgestaltete Anschlussadapter ist zu einer Vielzahl von unterschiedlich ausgestalteten Spülgütern kompatibel. Denn in Abkehr zum Stand der Technik findet keine Verschraubung zwischen Spülgut und Anschlussadapter statt. Der nach der Erfindung vorgesehene Anschlussadapter stellt vielmehr eine Spülkammer bereit, in die das Spülgut im bestimmungsgemäßen Verwendungsfall eingesetzt ist. Insoweit kommt es auf eine vom Spülgut bereitgestellte Anschlussgeometrie nicht weiter an.

Von Vorteil der erfindungsgemäßen Ausgestaltung ist ferner, dass nicht nur eine Innenbeaufschlagung des Spülguts mit Spülflotte stattfindet, sondern auch eine Außenbeaufschlagung. Im bestimmungsgemäßen Verwendungsfall wird die Spülkammer mit Spülflüssigkeit geflutet, so dass einerseits eine gründliche Außenreinigung als auch eine gründliche Innenreinigung des Spülgutes stattfindet, womit auch am Spülgut durch Antrocknung anhaftende Partikel gut abgereinigt werden können.

Der erfindungsgemäße Anschlussadapter ist universell einsetzbar. Er ist nicht auf bestimmte herstellertypische und/oder -spezifische Anschlussgeometrien beispielsweise hinsichtlich Durchmesser und/oder Gewindesteigung des Anschlussgewindes beschränkt. Es bedarf deshalb auch keiner auf die spezielle Anschlussgeometrie abgestimmten Spezial-Anschlussadapter. Der nach der Erfindung vorgesehene Anschlussadapter lässt sich vielmehr herstellerunabhängig für eine Vielzahl von in ihrer Anschlussgeometrie unterschiedlich ausgebildeten Spülgütern gleichermaßen einsetzen.

Zudem erlaubt der erfindungsgemäße Anschlussadapter eine vereinfachte Handhabung. Denn es bedarf im Unterschied zur Ausgestaltung nach dem Stand der Technik keiner Verschraubung mehr zwischen Anschlussadapter einerseits und zu reinigendem Spülgut andererseits. Das Spülgut kann vielmehr in einfacher Weise in die vom Anschlussadapter bereitgestellte Spülkammer eingesetzt werden. Alsdann ist verwenderseitig nur noch das Verschlussteil in seine Verschlussstellung zu bewegen, so dass das Spülgut während eines Spülvorgangs nicht in unbeabsichtigter Weise aus der Spülkammer hinausbewegt wird. Nach erfolgreich durchgeführter Reinigung ist das Verschlussteil zurück in seine Beschickungsstellung zu bewegen, was es verwenderseitig gestattet, das gereinigte Spülgut der Spülkammer des Anschlussadapters zu entnehmen.

Im Ergebnis der erfindungsgemäßen Ausgestaltung ist ein Anschlussadapter geschaffen, der universell einsetzbar ist, eine vereinfachte Handhabung ermöglicht und darüber hinaus nicht nur eine Innen-, sondern auch eine Außenreinigung ermöglicht.

Es ist gemäß einem weiteren Merkmal der Erfindung vorgesehen, dass der Grundkörper des Anschlussadapters anschlussstutzenseitig einen Gewindeabschnitt aufweist. Dies gestattet es, den Grundkörper mit einem Anschlussstutzen der Rohrleitung durch Verschrauben zu verbinden. Dabei ist der Gewindeabschnitt in Entsprechung des Anschlussstutzens standardisiert ausgebildet und verfügt beispielsweise über ein Gewinde der Größe M8. Typischerweise stellt der Gewindeabschnitt ein Außengewinde bereit, wohingegen der Anschlussstutzen der Rohrleitung ein Innengewinde aufweist.

Gemäß einem weiteren Merkmal der Erfindung ist vorgesehen, dass der Grundkörper einen im Querschnitt kreisförmigen Zylinderabschnitt aufweist, der die Spülkammer beherbergt. Der Grundkörper verfügt neben dem schon vorbeschriebenen Gewindeabschnitt über einen im Querschnitt kreisförmigen Zylinderabschnitt. Dieser Zylinderabschnitt ist als Hohlzylinder ausgebildet, wobei der Hohlraum die Spülkammer bildet. Für einen strömungstechnischen Anschluss der Spülkammer an den Anschlussstutzen beherbergt der Grundkörper einen Innenkanal, durch den hindurch im bestimmungsgemäßen Verwendungsfall Spülflüssigkeit bis in die Spülkammer einströmt.

Gemäß einem weiteren Merkmal der Erfindung ist vorgesehen, dass die Spülkammer in eine stirnseitige Bohrung des Grundkörpers mündet. Im bestimmungsgemäßen Verwendungsfall strömt über die Bohrung in die Spülkammer zuvor eingebrachte Spülflüssigkeit ab. Die Spülkammer kann mithin von Spülflüssigkeit bei gleichzeitigem Zu-und Ablauf durchströmt werden, so dass im Ergebnis eine Außen- und Innenreinigung eines von der Spülkammer aufgenommenen Spülguts stattfindet.

Gemäß einem weiteren Merkmal der Erfindung ist vorgesehen, dass die Beschickungsöffnung in einem die Spülkammer radial begrenzenden Wandabschnitt des Grundkörpers ausgebildet ist. Die Spülkammer ist mithin über eine seitliche Öffnung zugänglich. Durch diese Öffnung wird im bestimmungsgemäßen Verwendungsfall das zu reinigende Spülgut in die Spülkammer eingebracht.

Bei dem verdrehbar am Grundkörper angeordneten Verschlussteil handelt es sich gemäß einem weiteren Merkmal der Erfindung um eine Hülse. Diese Hülse ist verdrehbar am Grundkörper gelagert und kann relativ zum Grundkörper verdreht werden, welcher Grundkörper aufgrund seiner Anordnung am Anschlussstutzen feststehend ausgebildet ist. Dabei kann die Hülse aus einer Verschlussstellung in eine Beschickungsstellung und umgekehrt überführt werden. In der Beschickungsstellung ist die Beschickungsöffnung zugänglich, das heißt in die vom Grundkörper bereitgestellte Spülkammer kann zu reinigendes Spülgut eingesetzt werden. In der Verschlussstellung der Hülse ist die Beschickungsöffnung indes verschlossen, so dass im bestimmungsgemäßen Reinigungsfall ein Herausbewegen des Spülguts aus der Spülkammer sicher vermieden ist.

Gemäß einem weiteren Merkmal der Erfindung ist vorgesehen, dass die Wandung der Hülse eine korrespondierend zur Beschickungsöffnung ausgebildeten Durchbruch aufweist. Sofern sich die Hülse in ihrer Beschickungsstellung befindet, sind die vom Grundkörper bereitgestellte Beschickungsöffnung sowie der von der Hülse bereitgestellte Durchbruch zumindest abschnittsweise deckungsgleich zueinander ausgerichtet. Es ist so verwenderseitig gestattet, zu reinigendes Spülgut in bestimmungsgemäßer Weise in die Spülkammer einzubringen. In Verschlussstellung der Hülse ist diese im Vergleich zur Beschickungsstellung relativ gegenüber dem Grundkörper verdreht, so dass nicht mehr der von der Hülse bereitgestellte Durchbruch, sondern ein Wandabschnitt der Hülse in Überdeckung mit der Beschickungsöffnung liegt, womit diese verschlossen ist.

Gemäß einem weiteren Merkmal der Erfindung ist vorgesehen, dass die Hülse dem Gewindeabschnitt des Grundkörpers gegenüberliegend einen vorzugsweise einstückig mit der Hülse ausgebildeten Deckel trägt. Der Deckel schließt die Hülse mithin nach oben hin ab. Dabei stellt der Deckel eine zumindest abschnittsweise deckungsgleich zur Bohrung des Grundkörpers ausgebildete Öffnung bereit. In die Spülkammer eingebrachte Spülflüssigkeit kann mithin durch die Bohrung des Grundkörpers und die Öffnung des Deckels abströmen. Zudem kann im bestimmungsgemäßen Reinigungsfall das Spülgut zumindest abschnittsweise aus der Spülkammer durch die Bohrung des Grundkörpers und die Öffnung des Deckels hinausragen.

Gemäß einem weiteren Merkmal der Erfindung ist eine Arretiereinrichtung vorgesehen, mittels welcher der Grundkörper und die Hülse in Verschlussstellung der Hülse lagegesichert sind. Mittels der Rasteinrichtung ist sichergestellt, dass sich die Hülse nicht ungewollt aus der Verschlussstellung in die Beschickungsstellung bewegt.

Die Hülse und der Grundkörper sind gemäß einem weiteren Merkmal der Erfindung unverlierbar aneinander angeordnet. Die Handhabung des erfindungsgemäßen Anschlussadapters wird damit weiter erleichtert, denn kann er verwenderseitig als quasi einstückiges Bauteil gehandhabt werden. Es besteht jedenfalls auch im bestimmungsgemäßen Verwendungsfall nicht die Gefahr, dass sich Hülse und Grundkörper voneinander trennen.

Gemäß einer weiter bevorzugten Ausführungsform der Erfindung ist vorgesehen, dass die Hülse eine Führungsnut aufweist, in die ein am Grundkörper angeordneter Führungsstift eingreift. Durch diese Ausgestaltung wird ein Formschluss zwischen Grundkörper einerseits und Hülse andererseits geschaffen, womit die unverlierbare Anordnung von Hülse und Grundkörper realisiert ist. Zudem ist mittels dieser Ausgestaltung sichergestellt, dass definierte Verdrehanschläge gegeben sind, wobei der eine Verdrehanschlag die Offen- bzw. Beschickungsstellung und der andere Verdrehanschlag die Verschlussstellung definiert.

Die Führungsnut weist gemäß einem weiteren Merkmal der Erfindung eine Aussparung aus, die sich quer zur Längserstreckung der Führungsnut erstreckt. Dabei ist die Aussparung korrespondierend zum Führungsstift ausgebildet, so dass dieser bei entsprechender Verdrehstellung der Hülse relativ gegenüber dem Grundkörper in der Aussparung zu liegen kommt. Auf diese Weise ist eine Arretiereinrichtung geschaffen, die bei gleichzeitig einfacher Handhabbarkeit eine effektive Lagesicherung gewährleistet.

Weitere Merkmale und Vorteile der Erfindung ergeben sich aus der nachfolgenden Beschreibung anhand der Figuren. Dabei zeigen:
- Fig. 1.: in schematisch perspektivischer Darstellung einen Anschlussadapter nach der Erfindung;
- Fig. 2: in schematischer Seitenansicht den Anschlussadapter nach Figur 1;
- Fig. 3: in einer Draufsicht von oben den Anschlussadapter nach Figur 1;
- Fig. 4: in schematischer Seitenansicht den erfindungsgemäßen Anschlussadapter in Beschickungsstellung;
- Fig. 5: den Anschlussadapter nach Figur 4 in etwas gedrehter Seitenansicht;
- Fig. 6: in einer weiteren schematischen Seitenansicht den Anschlussadapter nach der Erfindung;
- Fig. 7: in geschnittener Darstellung den Anschlussadapter nach der Erfindung gemäß Schnittlinie B-B nach Figur 6;
- Fig. 8: in schematischer Darstellung einen an eine Rohrleitung angeschlossenen Anschlussadapter und
- Fig. 9: in einer Ausschnittsdarstellung eine Detailansicht des Anschlussadapters nach Figur 8.

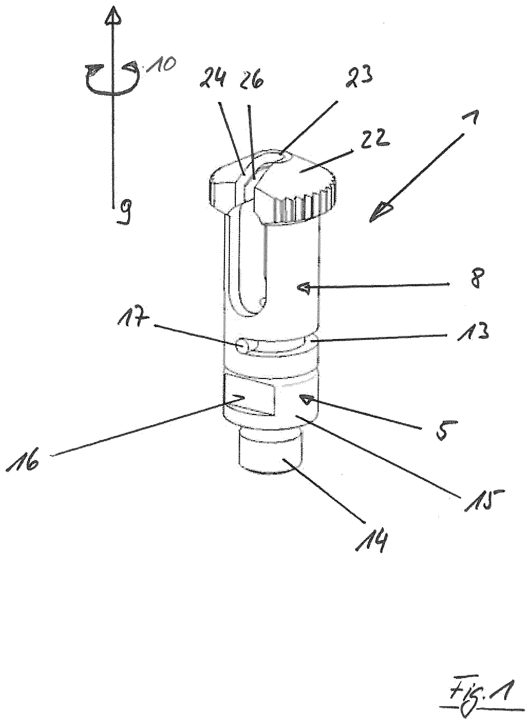

Figur 1 lässt in schematischer Perspektivdarstellung einen Anschlussadapter 1 nach der Erfindung erkennen.

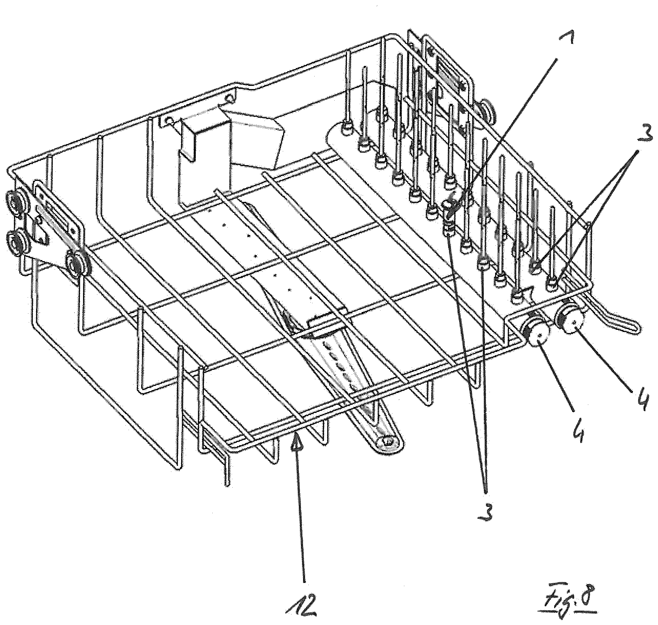

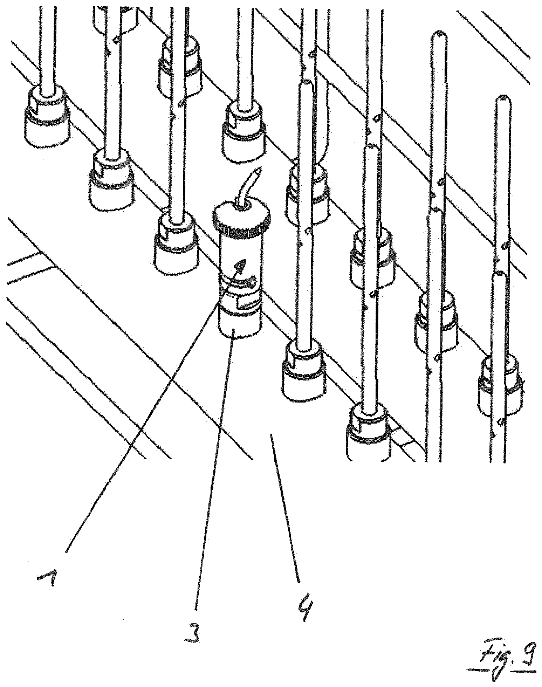

Wie sich aus einer Zusammenschau insbesondere der Figuren 8 und 9 ergibt, dient der Anschlussadapter 1 dem strömungstechnischen Anschluss eines Spülguts 2 an einen Anschlussstutzen 3 einer Rohrleitung 4, die im bestimmungsgemäßen Reinigungsfall mit Spülflüssigkeit beaufschlagt wird. Als Spülgut 2 kommt insbesondere dentales Spülgut in Betracht, so zum Beispiel eine Ultraschallspitze 2, wie sie in den Figuren 8 und 9 dargestellt ist.

Wie die Darstellung nach den Figuren 8 und 9 ferner erkennen lässt, ist die Rohrleitung 4 im gezeigten Ausführungsbeispiel als Teil eines Spülkorbs 12 ausgebildet, der in den Spülraum eines in den Figuren nicht näher dargestellten Reinigungs- und/oder Desinfektionsautomaten verbracht werden kann. Dabei sind je Rohrleitung 4 eine Mehrzahl von Anschlussstutzen 3 vorgesehen, wobei je Anschlussstutzen 3 ein Anschlussadapter 1 nach der Erfindung vorgesehen sein kann. Der besseren Übersicht wegen ist in Figuren 8 und 9 jeweils nur ein Anschlussadapter 1 dargestellt.

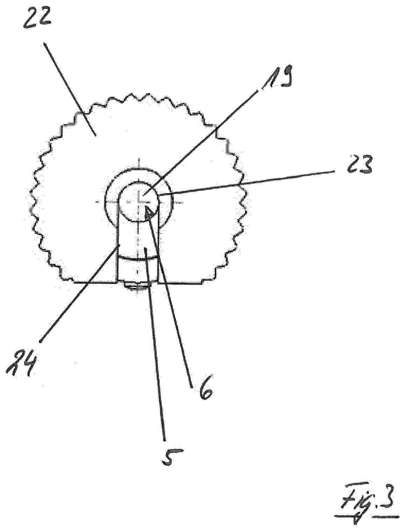

Der konstruktive Aufbau des erfindungsgemäßen Anschlussadapters 1 ergibt sich aus einer Zusammenschau der Figuren 1 bis 7.

Wie den Figuren zu entnehmen ist, verfügt ein Anschlussadapter 1 über einen Grundkörper 5 sowie über ein bewegbar am Grundkörper 5 angeordnetes Verschlussteil 8. Im gezeigten Ausführungsbeispiel ist das Verschlussteil 8 als Hülse ausgebildet, das oberseitig mit einem Deckel 22 abschließt.

Der Grundkörper 5 verfügt über einen Gewindeabschnitt 14. Dieser ist mit einem Außengewinde ausgerüstet und dient zum Anschluss an einen Anschlussstutzen 3 einer Rohrleitung 4, zu welchem Zweck der Anschlussstutzen 3 über ein entsprechend ausgebildetes Innengewinde verfügt. Desweiteren verfügt der Grundkörper 5 über einen Werkzeugabschnitt 15, der Werkzeugflächen 16 bereitstellt. Für ein Aufschrauben des Anschlussadapters 1 auf einen Anschlussstutzen 3 kann ein entsprechendes Werkzeug, beispielsweise ein Maulschlüssel in Eingriff mit dem Werkzeugabschnitt 15 gebracht werden.

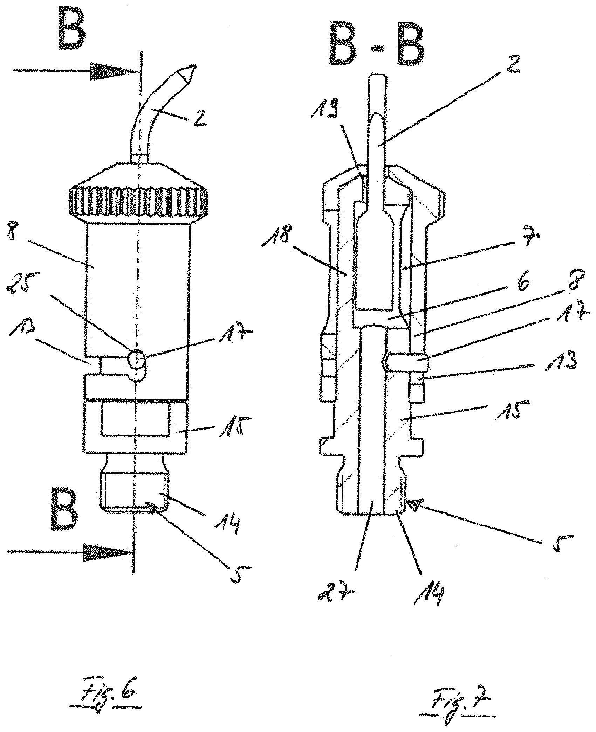

In Höhenrichtung des Anschlussadapters 1 folgt dem Werkzeugabschnitt 15 ein Zylinderabschnitt 18 nach. Dieser Zylinderabschnitt 18 ist als Hohlzylinder ausgebildet, wobei der vom Zylinderabschnitt bereitgestellte Hohlraum eine Spülkammer 6 definiert. Dieser Sachzusammenhang ergibt sich insbesondere aus der Schnittdarstellung nach Figur 7.

Wie sich aus der Schnittdarstellung nach Figur 7 ferner ergibt, mündet die Spülkammer 6 oberseitig in eine Bohrung 19 ein. Für den strömungstechnischen Anschluss an einen Anschlussstutzen 3 einer Rohrleitung 4 dient ein vom Grundkörper 5 bereitgestellter Innenkanal 27. Durch diesen strömt im bestimmungsgemäßen Verwendungsfall Spülflotte in die Spülkammer 6 ein, die die Spülkammer 6 nach einem Durchströmen durch die Bohrung 19 verlassen kann.

Zur Beschickung der Spülkammer 6 mit einem zu reinigenden Spülgut 2 ist in einem die Spülkammer 6 radial begrenzenden Wandabschnitt 20 des Grundkörpers 5 eine Beschickungsöffnung 7 ausgebildet. Durch diese hindurch ist das Spülgut 2 verwenderseitig in die Spülkammer 6 einzubringen.

Die Hülse 8 ist verdrehbar vom Grundkörper 5 gehalten, wobei eine Verdrehung der Hülse 8 in Entsprechung des Pfeils 10 um die Hochachse 9 gestattet ist, wie sich dies insbesondere aus der Darstellung nach Figur 1 ergibt.

Die Hülse 8 und der Grundkörper 9 sind unverlierbar miteinander gekoppelt. Zu diesem Zweck verfügt die Hülse 8 über eine Führungsnut 13, in die ein am Grundkörper 5 angeordneter Führungsstift 17 eingreift. Montageseits wird der Stift 17 in eine dafür vorgesehene Bohrung des Grundkörpers 5 eingepresst, nachdem die Hülse 8 auf den Grundkörper 5 aufgesetzt ist. Auf diese Weise wird eine formschlüssige Kopplung von Hülse 8 und Grundkörper 5 erreicht, so dass diese unverlierbar aneinander gekoppelt sind.

Die Führungsnut 13 stellt zudem Verdrehanschläge zur Verfügung, so dass die Hülse 8 in definierte Endlagen relativ gegenüber dem Grundkörper 5 verbracht werden kann.

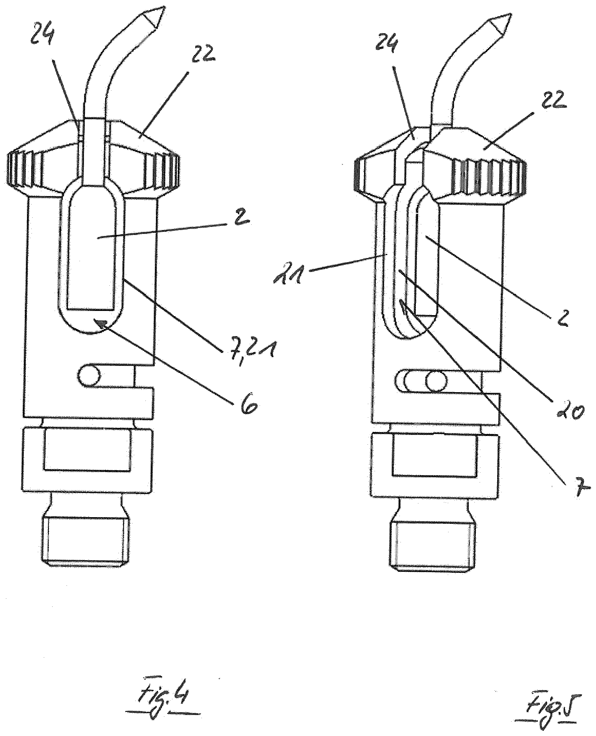

Die Hülse 8 verfügt über einen seitlich ausgebildeten Durchbruch 21, wie dies insbesondere aus einer Zusammenschau der Figuren 4 und 5 zu erkennen ist. Die Hülse 8 kann aufgrund ihrer verdrehbaren Anordnung aus einer Verschlussstellung in eine Beschickungsstellung und umgekehrt überführt werden, wobei die grundkörperseitige Beschickungsöffnung 7 und der hülsenseitige Durchbruch 21 in Beschickungsstellung zumindest abschnittsweise deckungsgleich zueinander ausgerichtet sind, wie dies die Figuren 4 und 5 erkennen lassen. In dieser Stellung der Hülse 8, das heißt der Beschickungsstellung ist ein verwenderseitiger Zugriff auf das von der Spülkammer 6 beherbergte Spülgut 2 gestattet.

In der Verschlussstellung der Hülse 8, wie sie beispielsweise in den Figuren 6 und 7 gezeigt ist, ist die Hülse 8 in Entsprechung des Pfeils 10 soweit gegenüber dem Grundkörper 5 verdreht, dass die Beschickungsöffnung 7 und der Durchbruch 21 nicht mehr einander überdecken. Die Beschickungsöffnung 7 ist vielmehr durch den Durchbruch frei ausgebildeten Wandabschnitt der Hülse 8 verschlossen. In dieser Stellung ist ein unbeabsichtigtes Hinausbewegen des Spülguts 2 aus der Spülkammer 6 durch die Beschickungsöffnung 7 hindurch unterbunden.

Die Nut 13 verfügt über eine Aussparung 25, die sich quer zur Längserstreckung der Nut 13 erstreckt, wie sich die insbesondere aus der Darstellung nach Figur 6 ergibt. Dabei ist die Aussparung 25 korrespondierend zum Führungsstift 17 ausgebildet, so dass dieser in Verschlussstellung der Hülse 8 in die Aussparung 5 einwandern kann. Es ist so eine Arretierung der Hülse relativ gegenüber dem Grundkörper 5 gegeben, so dass die Hülse 8 nicht ungewollt aus der Verschlussstellung in die Beschickungsstellung verdrehen kann. Um eine Überführung der Hülse 8 in die Beschickungsstellung zu bewirken ist die Hülse 8 verwenderseitig in Richtung der Hochachse 9 zu verfahren und alsdann zu verdrehen, wobei die Verdrehfreiheit der Hülse 8 dann gegeben ist, wenn der Führungsstift 27 aus der Aussparung 25 hinausgewandert und in die Führungsnut 13 eingetaucht ist.

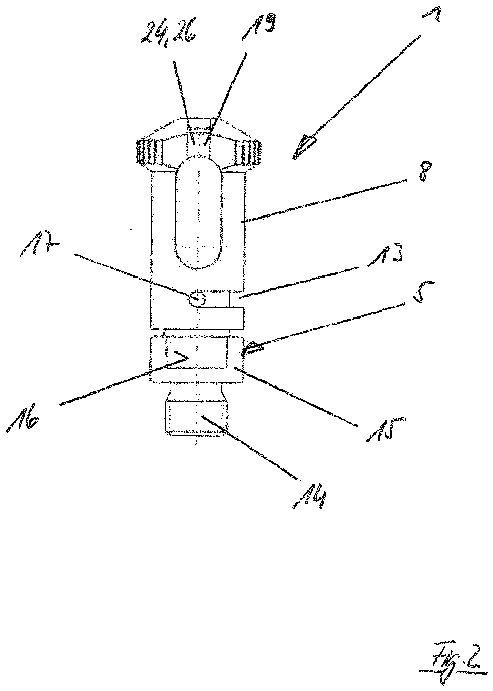

Bei dem in den Figuren gezeigten Spülgut 2 handelt es sich um eine mit einem Lumen ausgestattete Ultraschallspitze, die im bestimmungsgemäßen Reinigungsfall von der Spülkammer 6 zumindest abschnittsweise aufgenommen ist. Die eigentliche Spitze der Ultraschallspitze 2 ragt im Reinigungsfall durch die Bohrung 19 des Grundkörpers 5 und die Öffnung 23 der Hülse 8 hindurch. Um ein Einführen der Ultraschallspitze 2 in die Spülkammer 6 zu ermöglichen, sind sowohl die Bohrung 19 mit einem Einführschlitz 26 als auch die Öffnung 23 mit einem Einführschlitz 24 ausgerüstet, wie sich dies beispielsweise aus den Figuren 1 und 2 ergibt.

Der erfindungsgemäße Anschlussadapter 1 ist in vielerlei Hinsicht gegenüber vorbekannten Anschlussadaptern von Vorteil. Zum einen wird im Reinigungsfall durch Fluten der Spülkammer 6 mit Spülflüssigkeit eine Innenreinigung wie auch eine Außenreinigung des Spülguts 2 bewirkt. Ferner erlaubt der erfindungsgemäße Anschlussadapter eine verwenderseitig einfache Handhabung, da es im Unterschied zum Stand der Technik keiner Verschraubung des zu reinigenden Spülgutes 2 zum Anschluss an den Anschlussadapter 1 bedarf. Es ist vielmehr lediglich die Hülse 8 in ihre Beschickungsöffnung zu verdrehen, was dann ein einfaches Einsetzen des zu reinigenden Spülguts 2 in die Spülkammer 6 gestattet.

Darüber hinaus ist von Vorteil, dass der Anschlussadapter 1 hinsichtlich der Ausgestaltung des Spülguts 2 universell ausgebildet ist, da Spülgüter 2 unterschiedlichster Anschlussgeometriem, das heißt herstellerunabhängig gleichermaßen mit dem erfindungsgemäßen Anschlussadapter 1 an einen Anschlussstutzen 3 einer Rohrleitung, auch Injektorleiste genannt, angeschlossen werden können.

**Bezugszeichen**

| | | | |
|---|---|---|---|
| 1 | Anschlussadapter | 19 | Bohrung |
| 2 | Spülgut (Ultraschallspitze) | 20 | Wandabschnitt |
| 3 | Anschlussstutzen | 21 | Durchbruch |
| 4 | Rohrleitung | 22 | Decke |
| 5 | Grundkörper | 23 | Öffnung |
| 6 | Spülkammer | 24 | Schlitz |
| 7 | Beschickungsöffnung | 25 | Aussparung |
| 8 | Verschlussteil (Hülse) | 26 | Schlitz |
| 9 | Hochachse | 27 | Kanal |
| 10 | Pfeil | | |
| 12 | Spülkorb | | |
| 13 | Führungsnut | | |
| 14 | Gewindeabschnitt | | |
| 15 | Werkzeugabschnitt | | |
| 16 | Werkzeugfläche | | |
| 17 | Führungsstift | | |
| 18 | Zylinderabschnitt | | |

## Patentansprüche

1. Anschlussadapter für den strömungstechnischen Anschluss eines insbesondere dentalen Spülguts (2) an einen Anschlussstutzen (3) einer der Zuführung einer Spülflüssigkeit dienenden Rohrleitung (4), mit einem Grundkörper (5), der eine Spülkammer (6) zur zumindest teilweisen Aufnahme des Spülguts (2) aufweist, wobei der Grundkörper (5) zur Beschickung der Spülkammer (6) mit dem Spülgut (2) eine Beschickungsöffnung (7) bereitstellt, und mit einem bewegbar am Grundkörper (5) angeordneten Verschlussteil (8), mittels dem die Beschickungsöffnung (7) zumindest teilweise verschließbar ist,
**dadurch gekennzeichnet,**
**dass** die Beschickungsöffnung (7) in einem die Spülkammer (6) radial begrenzenden Wandabschnitt (20) des Grundkörpers (5) ausgebildet ist,
und **dass** das Verschlussteil (8) eine verdrehbar am Grundkörper (5) angeordnete Hülse (8) ist, deren Wandung einen korrespondierend zur Beschickungsöffnung (7) ausgebildeten Durchbruch (21) aufweist.

2. Anschlussadapter nach Anspruch 1, **dadurch gekennzeichnet, dass** der Grundkörper (5) anschlussstutzenseitig einen Gewindeabschnitt (14) aufweist.

3. Anschlussadapter nach Anspruch 1 oder 2, **dadurch gekennzeichnet, dass** der Grundkörper (5) einen im Querschnitt kreisförmigen Zylinderabschnitt (18) aufweist, der die Spülkammer (6) beherbergt.

4. Anschlussadapter nach einem der vorhergehenden Ansprüche, **dadurch gekennzeichnet, dass** die Spülkammer (6) in eine stirnseitige Bohrung (19) des Grundkörpers (5) mündet.

5. Anschlussadapter nach einem der vorhergehenden Ansprüche, **dadurch gekennzeichnet, dass** die Hülse (8) aus einer Verschlussstellung in eine Beschickungsstellung und umgekehrt überführbar ist, wobei die grundkörperseitige Beschickungsöffnung (7) und der hülsenseitige Durchbruch (21) in Beschickungsstellung zumindest abschnittsweise deckungsgleich ausgerichtet sind.

6. Anschlussadapter nach einem der vorhergehenden Ansprüche, **dadurch gekennzeichnet, dass** die Hülse (8) dem Gewindeabschnitt (14) des Grundkörpers (5) gegenüberliegend einen vorzugsweise einstückig mit der Hülse (8) ausgebildeten Deckel (22) trägt.

7. Anschlussadapter nach Anspruch 6, **dadurch gekennzeichnet, dass** der Deckel (22) eine zumindest abschnittsweise deckungsgleich zur Bohrung (19) des Grundkörpers (5) ausgebildete Öffnung (23) bereitstellt.

8. Anschlussadapter nach einem der vorhergehenden Ansprüche, **gekennzeichnet durch** eine Arretiereinrichtung, mittels welcher der Grundkörper (5) und die Hülse (8) in Verschlussstellung der Hülse (8) lagegesichert sind.

9. Anschlussadapter nach einem der vorhergehenden Ansprüche, **dadurch gekennzeichnet, dass** die Hülse (8) am Grundkörper (5) unverlierbar angeordnet ist.

10. Anschlussadapter nach einem der vorhergehenden Ansprüche, **dadurch gekennzeichnet, dass** die Hülse (8) eine Führungsnut (13) aufweist, in die ein am Grundkörper (5) angeordneter Führungsstift (17) eingreift.

11. Anschlussadapter nach Anspruch 10, **dadurch gekennzeichnet, dass** die Führungsnut (13) eine sich quer zur Längserstreckung der Führungsnut (13) erstreckende Aussparung (25) aufweist.

## Claims

1. Connection adapter for fluidically connecting an in particular dental item (2) to be rinsed to a connecting piece (3) of a pipeline (4) intended for supplying a rinsing fluid, comprising a main body (5) that comprises a rinsing chamber (6) for receiving the item (2) to be rinsed at least in part, the main body (5) providing a loading opening (7) for loading the rinsing chamber (6) with the item (2) to be rinsed, and comprising a closure part (8), arranged so as to be movable on the main body (5), by means of which the loading opening (7) can be closed, at least in part, **characterised in that** the loading opening (7) is formed in a wall portion (20) of the main body (5) that radially delimits the rinsing chamber (6), and **in that** the closure part (8) is a sleeve (8) arranged so as to be rotatable on the main body (5), the wall of which sleeve comprises a through hole (21) formed so as to correspond with the loading opening (7).

2. Connection adapter according to claim 1, **characterised in that** the main body (5) comprises a threaded portion (14) on the connecting piece side.

3. Connection adapter according to either claim 1 or claim 2, **characterised in that** the main body (5) has a cylindrical portion (18) which has a circular cross section and accommodates the rinsing chamber (6).

4. Connection adapter according to any of the preceding claims, **characterised in that** the rinsing chamber (6) opens into an end-face hole (19) in the main body (5).

5. Connection adapter according to any of the preceding claims, **characterised in that** the sleeve (8) can be transferred from a closed position into a loading position, and vice versa, the loading opening (7) in the main body and the through hole (21) in the sleeve being congruently aligned in the loading position, at least in portions.

6. Connection adapter according to any of the preceding claims, **characterised in that**, opposite the threaded portion (14) of the main body (5), the sleeve (8) supports a cover (22) that is preferably formed integrally with the sleeve (8).

7. Connection adapter according to claim 6, **characterised in that** the cover (22) provides an opening (23) that is formed so as to be congruent, at least in portions, with the hole (19) in the main body (5).

8. Connection adapter according to any of the preceding claims, **characterised by** a locking device, by means of which the main body (5) and the sleeve (8) are secured in position in the closed position of the sleeve (8).

9. Connection adapter according to any of the preceding claims, **characterised in that** the sleeve (8) is captively arranged on the main body (5).

10. Connection adapter according to any of the preceding claims, **characterised in that** the sleeve (8) has a guide groove (13) in which a guide pin (17) arranged on the main body (5) engages.

11. Connection adapter according to claim 10, **characterised in that** the guide groove (13) has a recess (25) extending transversely to the longitudinal extension of the guide groove (13).

## Revendications

1. Adaptateur de raccordement pour le raccordement fluidique d'un article à laver (2), en particulier dentaire, à un raccord (3) d'une conduite (4) servant à amener un liquide de lavage, comportant un corps de base (5) doté d'une chambre de lavage (6) destinée à recevoir au moins partiellement l'article à laver (2), le corps de base (5) comportant une ouverture de chargement (7) pour le chargement de l'article à laver (2) dans la chambre de lavage (6), et une pièce de fermeture (8) disposée de manière mobile sur le corps de base (5), laquelle pièce de fermeture permet de fermer au moins partiellement l'ouverture de chargement (7),
**caractérisé en ce que**
l'ouverture de chargement (7) est réalisée dans une section de paroi (20) du corps de base (5) qui délimite radialement la chambre de lavage (6), et **en ce que** la pièce de fermeture (8) est un manchon (8) disposé en rotation au niveau du corps de base (5), et dont la paroi comporte un passage (21) qui correspond à l'ouverture de chargement (7).

2. Adaptateur de raccordement selon la revendication 1, **caractérisé en ce que** le corps de base (5) du côté du raccord comporte une section filetée (14).

3. Adaptateur de raccordement selon la revendication 1 ou 2, **caractérisé en ce que** le corps de base (5) comporte une section cylindrique (18) circulaire en section transversale, dans laquelle la chambre de lavage (6) est logée.

4. Adaptateur de raccordement selon l'une des revendications précédentes, **caractérisé en ce que** la chambre de lavage (6) débouche dans un alésage (19) frontal du corps de base (5).

5. Adaptateur de raccordement selon l'une des revendications précédentes, **caractérisé en ce que** le manchon (8) peut être amené d'une position fermée à une position de chargement et vice versa, l'ouverture de chargement (7) du côté du corps de base et le passage (21) du côté du manchon étant alignés au moins par endroits dans la position de chargement.

6. Adaptateur de raccordement selon l'une des revendications précédentes, **caractérisé en ce que** le manchon (8) porte, en face de la section filetée (14) du corps de base (5), un couvercle (22) qui est de préférence réalisé d'une seule pièce avec le manchon (8).

7. Adaptateur de raccordement selon la revendication 6, **caractérisé en ce que** le couvercle (22) comporte une ouverture (23) qui est au moins partiellement congruente avec l'alésage (19) du corps de base (5).

8. Adaptateur de raccordement selon l'une des revendications précédentes, **caractérisé par** un dispositif de verrouillage, au moyen duquel le corps de base (5) et le manchon (8) sont fixés en position lorsque le manchon (8) est dans la position fermée.

9. Adaptateur de raccordement selon l'une des revendications précédentes, **caractérisé en ce que** le manchon (8) est disposé de manière imperdable au niveau du corps de base (5).

10. Adaptateur de raccordement selon l'une des revendications précédentes, **caractérisé en ce que** le manchon (8) comporte une rainure de guidage (13) dans laquelle une goupille de guidage (17) disposée au niveau du corps de base (5) vient en prise.

11. Adaptateur de raccordement selon la revendication 10, **caractérisé en ce que** la rainure de guidage (13) présente un évidement (25) s'étendant transversalement à l'extension longitudinale de la rainure de guidage (13).
